(19) Europäisches Patentamt
European Patent Office
Office européen des brevets

(11) **EP 0 905 256 B1**

(12) **FASCICULE DE BREVET EUROPEEN**

(45) Date de publication et mention
de la délivrance du brevet:
**04.08.2004   Bulletin 2004/32**

(51) Int Cl.[7]: **C12P 19/16**, C12P 19/22,
C13K 7/00

(21) Numéro de dépôt: **98402333.3**

(22) Date de dépôt: **23.09.1998**

(54) **Procédé de fabrication d'un sirop riche en maltose**

Verfahren zur Herstellung eines Siurps mit hohem Gehalt an Maltose

Process for producing a syrup rich in maltose

(84) Etats contractants désignés:
**AT BE CH DE DK ES FI FR GB GR IT LI NL PT SE**

(30) Priorité:  **26.09.1997  FR 9712036**

(43) Date de publication de la demande:
**31.03.1999   Bulletin 1999/13**

(73) Titulaire: **Roquette Frères**
**62136 Lestrem (FR)**

(72) Inventeurs:
• **Fouache, Catherine**
**62113 Sailly Labourse (FR)**
• **Delobeau, Didier**
**59660 Merville (FR)**
• **Quenon, Bruno**
**62840 Sailly sur la Lys (FR)**

(74) Mandataire: **Boulinguiez, Didier**
**Cabinet Plasseraud**
**65/67 rue de la Victoire**
**75440 Paris Cedex 09 (FR)**

(56) Documents cités:
**EP-A- 0 231 729**          **EP-A- 0 860 500**
**FR-A- 2 000 580**          **GB-A- 1 283 571**

• **JUNG K H ET AL: "Production of high
fructo-oligosaccharide syrup with two enzyme
system of fructosyltransferase and glucose
oxidase." BIOTECHNOLOGY LETTERS, vol. 15,
no. 1, 1993, pages 65-70, XP002065061**
• **PATENT ABSTRACTS OF JAPAN vol. 010, no.
287 (C-375), 30 septembre 1986 & JP 61 104794
A (KOHJIN CO LTD;OTHERS: 01), 23 mai 1986**

Remarques:
Le dossier contient des informations techniques
présentées postérieurement au dépôt de la
demande et ne figurant pas dans le présent
fascicule.

Printed by Jouve, 75001 PARIS (FR)

**Description**

L'invention concerne un procédé de fabrication d'un sirop riche en maltose. Elle concerne également un procédé de fabrication d'un sirop riche en maltitol à partir d'un sirop riche en maltose obtenu par le procédé conforme à la présente invention.

On connaît déjà des procédés permettant d'obtenir des sirops riches en maltose. Parmi ces procédés, on peut notamment citer celui décrit par HODGE et Coll. dans "Cereal Chemistry" n° 25, pages 19-30, Janvier 1948, et qui comprend une étape de précipitation des dextrines limites par des solutions alcooliques et celui décrit par WOLFROM et THOMPSON dans "Methods in carbohydrate chemistry", 1962, pages 334-335, et qui comprend une étape de cristallisation répétée de l'octaacétate de maltose suivie de la cristallisation du maltose.

D'autres procédés de fabrication de sirops riches en maltose ont encore été proposés comprenant une étape d'adsorption sur charbon des dextrines (US-A-4.194.623), une étape de chromatographie sur zéolithes ou résines cationiques ou anioniques (FR-A-2.510.581), une étape d'ultrafiltration des sirops de maltose (US-A-4.429.122), l'utilisation combinée de plusieurs enzymes différentes, à savoir une α-amylase, une β-amylase et une isoamylase ou une pullulanase (FR-A-2.012.831).

Cette dernière technique présente, par rapport aux précédentes, de nombreux avantages. Elle souffre néanmoins de certains inconvénients, dont notamment celui résidant dans le fait que les saccharifications doivent être effectuées à des teneurs en matières sèches très basses, de l'ordre de 20 g/l, pour obtenir une efficacité d'hydrolyse maximum des enzymes.

Le document FR-A-2.000.580 décrit un procédé de préparation d'un sirop à teneur élevée en maltitol par hydrogénation d'un sirop à teneur élevée en maltose qui est obtenu par liquéfaction d'un lait d'amidon à faible teneur en matières sèches jusqu'à un dextrose-équivalent inférieur à 2, le produit ainsi obtenu étant saccharifié sous l'action d'enzymes spécifiques.

Ce procédé est coûteux, d'un rendement médiocre et donne lieu à des problèmes de contamination bactérienne et à des phénomènes de rétrogradation de l'amylose. En outre, le sirop obtenu contient des proportions de polymères de degré de polymérisation (DP, dans la suite de la description) supérieur ou égal à 4, qui sont gênantes.

Le document EP 0 231 729 décrit un procédé de dégradation enzymatique du contenu en hydrates de carbone (amidon) de farine de céréales, qui met en oeuvre une α-amylase (Termamyl 120 L de NOVO) puis une β-amylase (dont l'action peut éventuellement être combinée à une enzyme de type pullulanase (NOVO's Promozyme 2004). Le procédé permet la production de maltose et de maltotriose.

Plus récemment, le document US-A-5.141.859 a proposé un procédé de fabrication d'un sirop à teneur élevée en maltose, mettant en oeuvre deux étapes successives de saccharification. Ce document préconise en effet un procédé comprenant une première étape de saccharification en présence d'une β-amylase et une étape subséquente de saccharification en présence d'une α-amylase maltogénique. Selon ce document, l'α-amylase maltogénique est utilisée, après la première étape de saccharification à la β-amylase, pour hydrolyser les oligosaccharides (de DP3 à DP7) et essentiellement le maltotriose (trisaccharide), en maltose et glucose.

De manière surprenante et inattendue, la Société Demanderesse a constaté que des sirops, à teneur aussi élevée en maltose que ceux décrits dans le document US-A-5.141.859, pouvaient être obtenus en saccharifiant un lait d'amidon liquéfié tout d'abord au moyen d'une α-amylase maltogénique puis en poursuivant cette saccharification au moyen d'une β-amylase.

Ainsi, contrairement à l'enseignement du document US-A-5.141.859, la société demanderesse a mis en évidence que l'α-amylase maltogénique était capable d'hydrolyser les polysaccharides d'un lait d'amidon liquéfié, et que cette enzyme pouvait donc être mise en oeuvre directement sur ce dernier sans passer d'abord par une étape de saccharification à la β-amylase.

Cette découverte est d'autant plus surprenante que l'on aurait pu penser que, après la saccharification à l'aide d'une α-amylase maltogénique, la poursuite de la saccharification à la β-amylase n'aurait aucun effet sur la richesse en maltose du sirop obtenu. En effet, l'α-amylase maltogénique est connue d'une part pour libérer de l'α-maltose et, d'autre part, pour être capable d'hydrolyser, à la différence de la β-amylase, le maltotriose en maltose et glucose. On pouvait donc s'attendre à ce que la mise en oeuvre de l'α-amylase maltogénique aurait permis, à elle seule, d'obtenir un hydrolysat à très haute teneur en maltose et que l'addition subséquente de β-amylase soit superflue. La société demanderesse a constaté, contre toute attente, que tel n'était pas le cas.

L'invention propose donc un procédé de fabrication d'un sirop riche en maltose, comprenant les étapes successives consistant à :

(a) effectuer une liquéfaction d'un lait d'amidon
(b) effectuer une saccharification du lait d'amidon liquéfié en présence d'une α-amylase maltogénique ;
(c) poursuivre la saccharification du lait d'amidon liquéfié en présence d'une β-amylase et d'au moins une enzyme débranchante choisie dans le groupe constitué par les pullulanases et les isoamylases en vue de l'obtention d'un sirop riche en maltose.

L'invention propose encore un procédé de fabrication d'un sirop riche en maltose, comprenant les étapes successives consistant à :

(a) effectuer une liquéfaction du lait d'amidon ;
(b) effectuer une saccharification du lait d'amidon liquéfié en présence d'une α-amylase maltogénique et d'au moins une enzyme débranchante choisie dans le groupe constitué par les pullulanases et les isoamylases, en vue de l'obtention d'un sirop riche en maltose.

On peut éventuellement poursuivre l'étape b de saccharification du lait d'amidon liquéfié en présence d'une β-amylase.

En poursuivant ses efforts de recherche sur les spécificités de l'α-amylase maltogénique, la Société Demanderesse a en outre constaté que si l'utilisation d'α-amylase maltogénique permettait effectivement et avantageusement d'abaisser la proportion en maltotriose par hydrolyse de ce dernier en maltose et glucose, elle présentait l'inconvénient majeur de générer des quantités importantes de glucose, et éventuellement de sorbitol en cas d'hydrogénation des hydrolysats. En effet, au glucose résiduel obtenu après saccharification du lait d'amidon liquéfié vient s'ajouter une proportion importante de glucose issu de l'hydrolyse du maltotriose par l'α-amylase maltogénique.

Ces quantités importantes de glucose, donc de sorbitol après hydrogénation, rendent la cristallisation du maltitol plus difficile et conduisent à une baisse de la richesse des cristaux, rendant ceux-ci mal adaptés à certaines applications comme par exemple la fabrication de chocolat.

En outre, la persistance de glucose ou de sorbitol libres dans les sirops de maltose ou de maltitol occasionnent d'autres inconvénients tels qu'un abaissement de la viscosité et de l'humidité relative d'équilibre des produits dans lesquels ils sont incorporés en tant que succédanés du sucre.

Consciente qu'il existe un intérêt croissant pour les produits à très haute teneur en maltose, la Société Demanderesse a conduit de nombreuses recherches dans le but de mettre au point un procédé économique et extrêmement fiable permettant d'obtenir de tels produits.

D'une manière extrêmement simple et particulièrement efficace au regard de tout ce qui a été proposé à ce jour, la Société Demanderesse a constaté que des sirops à très haute teneur en maltose pouvaient facilement être fabriqués en soumettant un sirop de maltose, obtenu conformément au procédé selon l'invention, à une étape supplémentaire de transformation du glucose à l'aide notamment d'une glucose oxydase, d'une levure ou d'une bactérie oxydante.

Malgré sa simplicité, cette étape supplémentaire du procédé conforme à l'invention permet de façon surprenante de fabriquer, avec un excellent rendement, un sirop à haute teneur en maltose sans mettre en oeuvre une étape de séparation coûteuse du type chromatographie par exemple comme c'est le cas des documents EP-A-185.595 et US-A-5.141.859.

La première étape du procédé conforme à l'invention est en soi connue. Elle consiste à liquéfier un lait d'amidon dont l'origine botanique peut être quelconque : il peut provenir du blé, du maïs ou de la pomme de terre par exemple.

Ce lait d'amidon ou de fécule est additionné d'acide dans le cas d'une liquéfaction dite acide, ou d'une α-amylase dans le cas d'une liquéfaction enzymatique.

Dans le procédé conforme à l'invention, on préfère effectuer une hydrolyse ménagée du lait d'amidon de façon à obtenir un lait d'amidon liquéfié à faible taux de transformation. Ainsi, les conditions de température, de pH, de taux d'enzyme et de calcium, connues de l'homme du métier, sont déterminées de manière telle qu'elles permettent d'obtenir un DE (Dextrose Equivalent) inférieur à 10, de préférence inférieur à 6, et plus particulièrement inférieur à 4.

De préférence, l'étape de liquéfaction est conduite en deux sous-étapes, la première consistant à chauffer, pendant quelques minutes et à une température comprise entre 105 et 108°C, le lait d'amidon en présence d'une α-amylase (type TERMAMYL[R] 120L commercialisée par la société NOVO) et d'un activateur à base de calcium, la seconde consistant à chauffer le lait d'amidon ainsi traité à une température comprise entre 95 et 100°C pendant une à deux heures.

Une fois l'étape de liquéfaction terminée, dans les conditions de teneur en matières sèches, de pH, de taux d'enzyme et de calcium bien connues de l'homme du métier, on procède à l'inhibition de l'α-amylase. Cette inhibition de l'α-amylase peut se faire de préférence par voie thermique, en procédant en sortie de liquéfaction à un choc thermique de quelques secondes à une température supérieure ou égale à 130°C.

On effectue ensuite l'étape de saccharification. Lors de cette étape, on soumet tout d'abord le lait d'amidon liquéfié à l'action d'une α-amylase maltogénique, telle que celle commercialisée par la société NOVO, sous le nom Maltogénase[R]. Lors de cette première étape de saccharification, l'α-amylase maltogénique peut être ajoutée en une seule fois ou en plusieurs fois.

On poursuit ensuite, après avoir laissé agir l'α-amylase maltogénique, la saccharification du lait d'amidon liquéfié au moyen d'une β-amylase telle que celle commercialisée par la société GENENCOR sous la dénomination SPEZYME[R] BBA 1500.

Lors de ces étapes, il convient d'associer aux enzymes ayant une activité maltogénique (α-amylase maltogénique et β-amylase) une enzyme hydrolysant spécifiquement les liaisons α-1,6 de l'amidon. Cet ajout d'une enzyme débranchante permet d'une part d'accélérer les réactions d'hydrolyse sans simultanément accélérer les réactions de reversion et, d'autre part, de réduire la quantité d'oligosaccharides hautement branchés résistant normalement à l'action des enzymes maltogéniques.

Cet ajout d'enzyme débranchante peut se faire, dans le procédé conforme à l'invention, au moment de l'ajout de l'α-amylase maltogénique ou au moment de l'ajout de la β-amylase.

La société Demanderesse a constaté que si l'on ajoute une enzyme débranchante au moment de l'ajout de l'α-amylase maltogénique, il est possible de ne pas mettre en oeuvre la β-amylase pour poursuivre l'étape de saccharification du lait d'amidon liquéfié.

Selon l'invention, l'enzyme débranchante est choisie dans le groupe constitué par les pullulanases et les isoamylases.

La pullulanase est, par exemple, celle commercialisée par la société ABM sous la dénomination PULLUZYME[R].

L'isoamylase est, par exemple, celle commercialisée par la société HAYASHIBARA.

Avantageusement, le procédé conforme à l'invention est mis en oeuvre en présence d'isoamylase pour laquelle la société demanderesse a constaté qu'elle permettait d'obtenir un sirop de maltose présentant une teneur en maltose plus élevée qu'en utilisant une pullulanase.

Dans un mode de réalisation particulier de l'invention, l'étape de saccharification peut être conduite également totalement ou partiellement en présence d'α-amylase fongique.

En fin de saccharification, il est possible d'ajouter un peu d'α-amylase, ce qui améliore généralement les étapes subséquentes de filtration. Les quantités et les conditions d'action des différentes enzymes mises en oeuvre dans les étapes de liquéfaction et de saccharification du lait d'amidon sont généralement celles qui sont recommandées pour l'hydrolyse de l'amidon et sont bien connues de l'homme du métier.

On effectue la saccharification jusqu'à ce que l'hydrolysat de maltose contienne au moins 87 % en poids de maltose et, de préférence, environ 90 % en poids de maltose. Elle dure environ 72 heures.

L'hydrolysat ainsi saccharifié est ensuite filtré sur filtre precoat ou par microfiltration sur membranes, puis déminéralisé.

A ce stade du procédé conforme à l'invention, il est éventuellement possible d'effectuer sur cet hydrolysat saccharifié et purifié, une cristallisation du maltose ou un tamisage moléculaire, ce tamisage moléculaire permettant d'enrichir l'hydrolysat en maltose. Cette étape de tamisage moléculaire peut permettre ainsi de récupérer :

- soit une première fraction enrichie en maltose et oligosaccharides supérieurs et une seconde fraction enrichie en glucose ;
- soit une première fraction enrichie en oligosaccharides supérieurs et une seconde fraction enrichie en maltose et glucose ;
- soit, enfin, une première fraction enrichie en oligosaccharides supérieurs, une deuxième fraction enrichie en maltose, et une troisième fraction enrichie en glucose.

Cette étape de tamisage moléculaire peut consister, par exemple, en une étape de séparation chromatographique ou en une étape de séparation sur membranes.

L'étape de fractionnement chromatographique est effectuée de manière connue en soi, de façon discontinue ou continue (lit mobile simulé), sur des adsorbants du type résines cationiques, ou sur des zéolithes fortement acides, chargées préférentiellement à l'aide d'ions alcalins ou alcalino-terreux tels que le calcium ou le magnésium mais plus préférentiellement à l'aide d'ions sodium.

En lieu et place de l'étape de séparation chromatographique, il est possible, dans le procédé conforme à l'invention, de mettre en oeuvre une étape de séparation par nanofiltration sur membranes. Des membranes de différents diamètres de pores sont fabriquées à partir de nombreux polymères et copolymères du type polysulfones, polyamides, polyacrylonitrates, polycarbonates, polyfuranes, etc.

Des exemples de l'utilisation de telles membranes sont décrits notamment dans les documents US-A-4.511.654, US-A-4.429.122 et WO-A-95/10627.

Selon un mode de réalisation avantageux du procédé conforme à l'invention, la partie non maltose issue des membranes ou de la chromatographie, comportant la fraction enrichie en glucose et/ou la fraction enrichie en oligosaccharides supérieurs, est recyclée en amont de l'étape de saccharification.

L'hydrolysat (ou sirop de maltose), ainsi obtenu, peut alors être soumis à une étape supplémentaire de transformation du glucose suivie d'une étape de désacidification sur échangeur d'anions. Cette étape supplémentaire peut être effectuée par oxydation enzymatique ou à l'aide d'une bactérie oxydante. Elle peut être aussi effectuée à l'aide de levures qui transforment le glucose en alcool qui s'élimine par évaporation dans la suite du procédé.

On préfère utiliser pour l'oxydation enzymatique, une composition enzymatique brute de glucose oxydase contenant aussi de la catalase.

La glucose oxydase catalyse la réaction suivante :

$$Glucose + O_2 + H_2O \rightarrow ACIDE\ GLUCONIQUE + H_2O_2$$

La catalase transforme l'eau oxygénée ainsi produite selon la réaction :

$$H_2O_2 \rightarrow H_2O + 1/2\ O_2$$

Une telle composition enzymatique est par exemple disponible auprès des établissements NOVO, DANEMARK sous la dénomination NOVOZYM 771.

Dans un mode de réalisation préféré du procédé conforme à l'invention, la glucose oxydase mise en oeuvre dans l'étape d'oxydation enzymatique est purifiée et, en particulier, débarrassée de son activité amyloglucosidase contaminante.

Une telle glucose oxydase est par exemple disponible auprès de la société FRIMOND sous la dénomination FRIMOX.

Cette oxydation enzymatique doit se dérouler dans un milieu aéré et le pH d'un tel milieu est de préférence maintenu à une valeur comprise entre 3,5 et 8,0, de préférence entre 4,0 et 7,0 et encore plus préférentiellement entre 5,0 et 6,0.

La concentration en hydrolysat de maltose n'est pas critique et peut varier de 5 à 75 %. Toutefois les concentrations élevées en hydrolysat imposent de travailler en régulant le pH à l'aide d'une base ou en effectuant l'oxydation en présence d'un sel tampon tel que le carbonate de calcium. Pour des raisons économiques, on préfère cependant effectuer l'oxydation sur des solutions aqueuses contenant environ 30 à 50 % de matières sèches. La température peut être ajustée dans une large gamme variant de 15 à 70°C mais, pour des raisons de commodité, on préfère travailler vers 30-40°C, températures auxquelles l'enzyme se montre plus stable.

Un matériel commode permettant d'effectuer cette oxydation consiste en un fermenteur aérobie, bien qu'il ne soit nullement nécessaire que cette étape se déroule dans des conditions stériles ni même rigoureuses d'aseptie. La quantité d'enzymes mise en oeuvre est telle que l'oxydation se déroule en 0,5 à 24 heures.

Lorsque l'on met en oeuvre, dans le procédé conforme à l'invention, une levure pour transformer le glucose résiduel, celle-ci appartient par exemple au genre *Saccaromyces.*

Lorsque l'on effectue l'étape supplémentaire de transformation du glucose résiduel au moyen d'une bactérie oxydante, celle-ci est choisie dans le groupe constitué par *Serratia, Pseudomonas, Gluconobacter, Acetobacter* et *Aspergillus.*

Pour ce qui concerne la seconde étape de désacidification, on préfère utiliser comme échangeur d'anions, une résine anionique forte qui permet de fixer efficacement les acides faibles que sont l'acide gluconique ou d'autres produits acides d'oxydation du glucose qui ont pu apparaître notamment lors du chauffage à haute température de celui-ci.

Les résines préférées sont celles qui portent des groupements fonctionnels du type amine quaternaire et encore plus préférentiellement des groupements triméthylamine quaternaire telles que la résine AMBERLITE IRA 900 commercialisée par ROHM and HAAS.

Ces résines sont utilisées sous leur forme hydroxyle ou base forte OH⁻.

Pour augmenter leur rendement de régénération avec les alcalis, on peut préférer les coupler avec une résine anionique faible essentiellement porteuse de groupements aminés tertiaires telle que l'AMBERLITE IRA 93 de la même société.

Grâce au procédé conforme à l'invention qui tire profit des bénéfices obtenus à la fois des étapes d'hydrolyse mises en oeuvre et des étapes d'oxydation et de désacidification, il est possible d'obtenir, avec des rendements supérieurs à 90 %, un hydrolysat d'amidon dont la teneur en maltose est supérieure à 95 %, et même supérieure à 98 % lorsqu'une isoamylase est mise en oeuvre dans les étapes d'hydrolyse.

L'hydrolysat de maltose obtenu conformément au procédé de l'invention peut alors être hydrogéné catalytiquement.

L'hydrogénation d'un tel hydrolysat s'effectue conformément aux règles de l'art qui conduisent par exemple à la production de sorbitol à partir du glucose.

On peut utiliser pour cette étape aussi bien des catalyseurs à base de ruthénium que des catalyseurs au nickel de RANEY. On préfère cependant utiliser des catalyseurs au nickel de RANEY qui sont moins onéreux.

Dans la pratique, on utilise de 1 à 10 % en poids de catalyseur par rapport à la matière sèche de l'hydrolysat soumis à l'hydrogénation. L'hydrogénation s'effectue de préférence sur un hydrolysat dont la matière sèche est comprise entre 15 et 50 %, dans la pratique voisine de 30 à 45 %, sous une pression d'hydrogène comprise entre 20 et 200 bars. Elle peut être effectuée de manière continue ou discontinue.

Lorsque l'on opère de manière discontinue, la pression d'hydrogène utilisée est généralement comprise entre 30 et 60 bars et la température à laquelle se déroule l'hydrogénation est comprise entre 100 et 150°C. On veille aussi à maintenir le pH du milieu d'hydrogénation par l'addition de soude ou de carbonate de sodium par exemple, mais sans dépasser un pH de 9,0. Cette manière de faire permet d'éviter l'apparition de produits de cracking ou d'isomérisation.

On arrête la réaction lorsque la teneur du milieu réactionnel en sucres réducteurs est devenue inférieure à 1 %, de préférence encore inférieure à 0,5 % et plus particulièrement inférieure à 0,1 %.

Après refroidissement du milieu réactionnel, on élimine le catalyseur par filtration et on déminéralise le sirop de maltitol ainsi obtenu sur des résines cationiques et anioniques. A ce stade, les sirops contiennent au moins 85 % de maltitol.

Selon une première variante du procédé conforme à l'invention, on met en oeuvre sur le sirop de maltitol obtenu à l'étape d'hydrogénation précédente, la succession des étapes, connue en elle-même (par exemple du document EP-A-189.704), consistant à :

- effectuer un fractionnement chromatographique, connu en soi, de manière à obtenir une fraction riche en maltitol ;
- concentrer la fraction riche en maltitol ;
- cristalliser et séparer les cristaux de maltitol formés ;
- recycler les eaux-mères de cristallisation en amont de l'étape de fractionnement chromatographique.

Une telle succession d'étapes permet d'augmenter les rendements spécifiques de cristallisation.

Selon une seconde variante du procédé conforme à l'invention, on met en oeuvre sur le sirop de maltitol obtenu à l'étape d'hydrogénation précédente, la succession des étapes suivantes consistant à :

- concentrer le sirop de maltitol ;
- cristalliser et séparer les cristaux de maltitol formés ;
- effectuer sur les eaux-mères de cristallisation un fractionnement chromatographique de manière à obtenir une fraction enrichie en maltitol et une fraction appauvrie en maltitol ;
- recycler la fraction enrichie en maltitol en amont de l'étape de cristallisation ;
- effectuer éventuellement sur la fraction appauvrie en maltitol, soit une hydrolyse acide, soit une hydrolyse enzymatique au moyen par exemple d'une amyloglucosidase immobilisée ou non ;
- éventuellement hydrogéner l'hydrolysat obtenu pour le transformer en un sirop de sorbitol.

Selon une autre variante du procédé conforme à l'invention, on met en oeuvre sur l'hydrolysat de maltose obtenu après saccharification la succession des étapes suivantes consistant à

- effectuer éventuellement un fractionnement chromatographique, connu en soi, de manière à obtenir une fraction riche en maltose et plus ou moins riche en maltotriose ;
- hydrogéner la fraction riche en maltose ;
- cristalliser et séparer les cristaux de maltitol formés.

D'autres caractéristiques et avantages de l'invention apparaîtront clairement à la lecture des exemples qui suivent. Ils ne sont toutefois donnés ici qu'à titre illustratif et non limitatif.

## EXEMPLE 1

Un lait d'amidon, constituée par une matière sèche de 31 %, est liquéfié de manière classique à l'aide de 0,2 % de TERMAMYL[R] 120L (α-amylase commercialisée par la société NOVO) à un pH de 5,7 à 6,5 jusqu'à un DE légèrement inférieur à 4.

On chauffe ensuite le milieu réactionnel pendant quelques secondes à 140°C de manière à inhiber l'α-amylase, puis on ajuste le pH entre 5 et 5,5 et la température à 55°C.

La saccharification est conduite à une matière sèche de 25 %, ou légèrement inférieure, d'abord en présence de pullulanase (PULLUZYME[R] 750L commercialisée par la société ABM) et d'α-amylase maltogénique (MALTOGENASE[R] 4000L commercialisée par la société NOVO) à des doses respectives de 0,1 % et 0,3 % par rapport à la matière sèche.

Après 48 heures de saccharification environ, mais en tout état de cause, dès que la teneur en maltose atteint ou dépasse 75 %, on ajoute 0,05 % de SPEZYME$^R$ DBA (commercialisée par la société GENENCOR) . La saccharification, qui dure environ 72 heures, donne un hydrolysat montrant la composition suivante, glucose : 6,6 %, maltose : 86,0 %, DP3 : 1,6 %, DP4 : 3,5 %, DP5 et + : 2,3 %.

L'hydrolysat subit ensuite une purification classique par filtration, décoloration et déminéralisation puis est concentré à environ 30 % de matière sèche.

## EXEMPLE 2

On effectue sur un lait d'amidon exactement les mêmes étapes de liquéfaction et de saccharification que celles décrites dans l'exemple 1 ci-dessus, excepté le fait que, lors de la saccharification, on utilise une isoamylase, et que le pH est maintenu à 4,7 pendant les 48 premières heures.

L'hydrolysat de maltose obtenu, au bout de 72 heures, montre la composition suivante, glucose : 7,2 %, maltose : 88,2 %, DP3 : 2,2 %, DP4 et + : 2,4 %.

## EXEMPLE 3

On effectue sur un lait d'amidon exactement les mêmes étapes de liquéfaction et de saccharification que celles décrites dans l'exemple 1 ci-dessus, excepté que l'on commence la saccharification avec la Maltogénase$^R$ seule et qu'après 48 heures, on ajoute la SPEZYME$^R$ DBA et la PULLUZYME$^R$ 750L.

L'hydrolysat de maltose obtenu au bout de 72 heures montre la composition suivante, glucose : 6,3 %, maltose : 84,1 %, DP3 : 1,8 %, DP4 : 2,6 %, DP5 et + : 5,2 %.

## EXEMPLE 4

La solution à 30 % d'hydrolysat de maltose obtenue dans l'exemple 1 ci-dessus est soumise à l'action de la glucose oxydase à raison de 0,7 %° de FRIMOX (commercialisée par la société FRIMOND) par rapport à la matière sèche en présence de 4,8 %° de catalase (commercialisée par la société BOEHRINGER) par rapport à la matière sèche.

Cette réaction se déroule en cuve aérée à raison de 1,5 volume d'air par volume de solution et par minute à un pH régulé de 6,0 par addition progressive de soude.

Elle a lieu à 35°C durant 7 heures au terme desquelles la teneur en glucose est inférieure à 0,5 %.

Cette solution est ensuite traitée sur une batterie de résines échangeuse d'ions comprenant en série une résine cationique forte IR200C puis une résine anionique forte IRA900. On met fin à cette déminéralisation lorsque la résistivité de la solution est tombée à une valeur inférieure à 10 000 ohms-cm.

L'hydrolysat montre alors la composition suivante, glucose : 0,5 %, maltose : 91,6 %, DP3 : 1,7 %, DP4 : 3,7 %, DP5 et + : 2,4 %.

## EXEMPLE 5

On effectue sur une solution à 30 % d'hydrolysat de maltose obtenu dans l'exemple 2 ci-dessus exactement la même succession d'étapes que celles décrites dans l'exemple 4 ci-dessus.

L'hydrolysat montre alors la composition suivante, glucose : 0,3 %, maltose : 94,7 %, DP3 : 2,4 %, DP4 et + : 2,6 %.

## EXEMPLE 6

L'hydrolysat de maltose de l'exemple 3 est placé dans un réacteur aérobie à 30°C. On ajoute de l'ammoniaque (à 20 % de concentration), à raison de 2,7 ml/litre de milieu dont 75 % sont ajoutés tout de suite et les 25 % restants après environ 1 heure de réaction. Le milieu est agité à 750 rpm, aéré à 1,2 vvm (volume/volume par minute) et additionné de 3,3 g/l de levures sèches (SAF-INSTANT commercialisée par S.I. LESAFFRE).

Après environ 12 heures de réaction, la composition obtenue est la suivante, glucose : 0,5 %, maltose : 89,3 %, DP3 : 1,9 %, DP4 : 2,8 %, DP5 et + : 5,5 %.

## EXEMPLE 7

*Serratia Marcescens* est conservée sous forme de tube congelé. Un tube sert à ensemencer 1,5 l de milieu de préculture contenant 50 g/l de glucose, 7 g/l d'extrait de levure, 5 g/l de corn steep et 5 g/l de carbonate de calcium. Cette préculture est mise à incuber 24 h à 30°C.

Elle est ensuite utilisée pour ensemencer 15 l de milieu de culture contenant l'hydrolysat de maltose obtenu dans l'exemple 3 (soit environ 130 g/l de maltose et 10 g/l de glucose), 4 g/l de phosphate d'ammonium, 0,4 g/l de sulfate de magnésium, 0,1 g/l de sulfate de fer et 10 g/l de carbonate de calcium. La culture est effectuée à 32°C, avec une agitation de 700 tours/mn et une aération de 1 vvm (volume/volume par minute) (15 litres d'air par minute).

Dans ces conditions, le glucose est entièrement consommé en 8 h de culture (transformé en acide gluconique et 2-cétogluconique) sans diminution de la teneur en maltose.

## EXEMPLE 8

L'hydrolysat de maltose obtenu dans l'exemple 1 ci-dessus est soumis à une étape de cristallisation du maltose de la manière suivante. On prépare une solution de maltose d'une richesse de 75 % en poids à une température de 75°C. On ensemence la solution de maltose avec 5 % en poids de germes de cristaux de maltose et refroidit la solution de 75°C à 40°C, à raison de 0,5°C par heure, en agitant la solution à 50 tours/min dans un cristallisoir à double paroi.

En fin de cristallisation, les cristaux sont séparés de la liqueur mère à l'aide d'une essoreuse centrifuge conventionnelle.

Le rendement de cristallisation est de 50 % en poids exprimé en poids de maltose cristallisé sur le poids de maltose de départ. La pureté en maltose des cristaux récupérés est de 97,5 % sur sec. La teneur en eau est de 5%.

## EXEMPLE 9

On procède à une étape de chromatographie continue de l'hydrolysat de maltose tel qu'obtenu dans l'exemple 1 ci-dessus de la manière suivante.

Quatre colonnes d'un litre de résine PCR 732 sodique thermostatées à 75°C sont assemblées en série et alimentées en continu avec l'hydrolysat de maltose amené à une richesse de 60 % en poids, à un débit de $110 \cdot 10^{-3}$ l/l.

En sortie de colonne on récupère les fractions enrichies en maltose de composition suivante, glucose : 6 %, maltose : 91,2 %, DP3 : 0,6 %, DP supérieur : 2,2 %.

Le rendement chromatographique en maltose est de 91,5 %.

## Revendications

**1.** Procédé de fabrication d'un sirop riche en maltose, comprenant les étapes successives consistant à :

(a) effectuer une liquéfaction d'un lait d'amidon ;
(b) effectuer une saccharification du lait d'amidon liquéfié en présence d'une α-amylase maltogénique ;
(c) poursuivre la saccharification du lait d'amidon liquéfié en présence d'une β-amylase et d'au moins une enzyme débranchante choisie dans le groupe constitué par les pullulanases et les isoamylases en vue de l'obtention d'un sirop riche en maltose.

**2.** Procédé de fabrication d'un sirop riche en maltose, comprenant les étapes successives consistant à :

(a) effectuer une liquéfaction du lait d'amidon ;
(b) effectuer une saccharification du lait d'amidon liquéfié en présence d'une α-amylase maltogénique et d'au moins une enzyme débranchante choisie dans le groupe constitué par les pullulanases et les isoamylases, en vue de l'obtention d'un sirop riche en maltose.

**3.** Procédé de fabrication d'un sirop riche en maltose selon la revendication 2, **caractérisé par le fait que** l'on poursuit l'étape b de saccharification du lait d'amidon liquéfié en présence d'une β-amylase.

**4.** Procédé selon la revendication 2, **caractérisé par le fait que** l'on effectue la saccharification en présence d'une α-amylase maltogénique et d'isoamylase.

**5.** Procédé selon l'une quelconque des revendications 1 à 4, **caractérisé par le fait que** l'on effectue la saccharification jusqu'à ce que le sirop de maltose contienne au moins 87 % et, de préférence, environ 90 % en poids de maltose.

6.  Procédé selon l'une quelconque des revendications 1 à 5, **caractérisé par le fait que** l'on soumet le sirop de maltose à une étape supplémentaire de transformation du glucose.

7.  Procédé selon la revendication 6, **caractérisé par le fait que** l'on effectue l'étape de transformation à l'aide d'un agent choisi dans le groupe comportant une glucose oxydase, une levure et une bactérie oxydante.

8.  Procédé selon la revendication 7, **caractérisé par le fait que** la glucose oxydase est purifiée.

9.  Procédé de fabrication d'un sirop riche en maltitol par hydrogénation d'un sirop riche en maltose, **caractérisé par le fait que** le sirop riche en maltose est obtenu par le procédé conforme à l'une quelconque des revendications 1 à 8.

**Patentansprüche**

1.  Verfahren zur Herstellung eines Sirups mit einem hohen Gehalt an Maltose, umfassend die aufeinanderfolgenden Schritte, die darin bestehen, daß:

    a) eine Verflüssigung einer Stärkemilch vorgenommen wird;
    b) eine Saccharifizierung der verflüssigten Stärkemilch in Gegenwart einer maltogenen $\alpha$-Amylase vorgenommen wird;
    c) die Saccharifizierung der verflüssigten Stärkemilch in Gegenwart einer $\beta$-Amylase und mindestens eines trennenden Enzyms weitergeführt wird, das aus der Gruppe ausgewählt wird, die aus Pullulanasen und Isoamylasen besteht, um einen Sirup mit hohem Maltosegehalt zu erhalten.

2.  Verfahren zur Herstellung eines Sirups mit hohem Gehalt an Maltose, umfassend die aufeinanderfolgenden Schritte, die darin bestehen, daß

    a) eine Verflüssigung der Stärkemilch vorgenommen wird;
    b) eine Saccharifizierung der verflüssigten Stärkemilch in Gegenwart einer maltogenen $\alpha$-Amylase und mindestens eines trennenden Enzyms vorgenommen wird, das aus der Gruppe ausgewählt wird, die aus Pullulanasen und Isoamylasen besteht, um einen Sirup mit hohem Maltosegehalt zu erhalten.

3.  Verfahren zur Herstellung eines Sirups mit einem hohen Gehalt an Maltose nach Anspruch 2, **dadurch gekennzeichnet, daß** man den Schritt b der Saccharifizierung der verflüssigten Stärkemilch in Gegenwart einer $\beta$-Amylase weiterführt.

4.  Verfahren nach Anspruch 2, **dadurch gekennzeichnet, daß** man die Saccharifizierung in Gegenwart einer maltogenen $\alpha$-Amylase und von Isoamylase durchführt.

5.  Verfahren nach einem der Ansprüche 1 bis 4, **dadurch gekennzeichnet, daß** man die Saccharifizierung durchführt, bis der Maltosesirup mindestens 87 Gew.-% und vorzugsweise etwa 90 Gew.-% Maltose enthält.

6.  Verfahren nach einem der Ansprüche 1 bis 5, **dadurch gekennzeichnet, daß** man den Maltosesirup einem zusätzlichen Schritt der Umwandlung der Glucose unterzieht.

7.  Verfahren nach Anspruch 6, **dadurch gekennzeichnet, daß** man den Umwandlungsschritt mit Hilfe eines Mittels durchführt, das aus der Gruppe ausgewählt wird, die eine Glucoseoxydase, eine Hefe und eine oxidierende Bakterie umfaßt.

8.  Verfahren nach Anspruch 7, **dadurch gekennzeichnet, daß** die Glucoseoxydase gereinigt wird.

9.  Verfahren zur Herstellung eines Sirups mit einem hohen Gehalt an Maltitol durch Hydrierung eines Sirups mit einem hohen Gehalt an Maltose, **dadurch gekennzeichnet, daß** der Sirup mit hohem Gehalt an Maltose nach dem Verfahren nach einem der Ansprüche 1 bis 8 erhalten wird.

**Claims**

1. Method of manufacturing a maltose-rich syrup, comprising the successive stages, consisting of:

   (a) carrying out a liquefaction of a starch milk;
   (b) carrying out a saccharification of the liquefied starch milk in the presence of a maltogenic $\alpha$-amylase;
   (c) continuing the saccharification of the liquefied starch milk in the presence of a $\beta$-amylase and at least one debranching enzyme chosen from the group consisting of pullulanases and isoamylases with a view to obtaining a syrup which is rich in maltose.

2. Method of manufacturing a maltose-rich syrup, comprising the successive stages, consisting of:

   (a) carrying out a liquefaction of the starch milk;
   (b) carrying out a saccharification of the liquefied starch milk in the presence of a maltogenic $\alpha$-amylase and at least one debranching enzyme chosen from the group consisting of pullulanases and isoamylases with a view to obtaining a syrup which is rich in maltose.

3. Method of manufacturing a maltose-rich syrup according to claim 2, **characterised by** the fact that stage (b) of saccharification of the liquefied starch milk is continued in the presence of a $\beta$-amylase.

4. Method according to claim 2, **characterised by** the fact that the saccharification is carried out in the presence of a maltogenic $\alpha$-amylase and of isoamylase.

5. Method according to any one of claims 1 and 2, **characterised by** the fact that the saccharification is carried out until the maltose syrup contains at least 87% and, by preference, about 90% by weight of maltose.

6. Method according to any one of claims 1 and 2, **characterised by** the fact that the maltose syrup is subjected to a supplementary stage of transformation of the glucose.

7. Method according to claim 6, **characterised by** the fact that the transformation stage is carried out with the aid of an agent chosen from the group comprising a glucose oxidase, a raising agent and an oxidising bacterium.

8. Method according to any one of claims 1 and 2, **characterised by** the fact that the glucose oxidase is purified.

9. Method of manufacturing a syrup which is rich in maltitol by hydrogenation of a maltose-rich syrup, **characterised by** the fact that the maltose-rich syrup is obtained by the method according to any one of claims 1 to 8.